# EUROPÄISCHE PATENTSCHRIFT

(11) **EP 2 178 825 B1**
(45) Veröffentlichungstag und Bekanntmachung des Hinweises auf die Patenterteilung: **11.03.2015**
(21) Anmeldenummer: 08784256.3
(22) Anmeldetag: 25.06.2008
(51) Int. Cl.: C07C 231/02, C07C 237/26, A61K 31/166, A61P 25/00

(54) **VERFAHREN ZUR SYNTHESE VON A-RING-AROMATISIERTEN ACETYL-MINOCYCLINEN**
METHOD FOR THE SYNTHESIS OF A-RING AROMATIZED ACETYL MINOCYCLINES
PROCÉDÉ DE SYNTHÈSE D'ACÉTYL-MINOCYCLINES AROMATISÉES AU NIVEAU DU CYCLE A

(30) Priorität: 20.07.2007 DE 102007034259
(43) Veröffentlichungstag der Anmeldung: 28.04.2010
(73) Patentinhaber: Otto-von-Guericke Universität Magdeburg Medizinische Fakultät, 39120 Magdeburg (DE)
(72) Erfinder: LORENZ, Peter, 39128 Magdeburg (DE); KREUTZMANN, Peter, 39116 Magdeburg (DE); ROTHE, Fritz, 39126 Magdeburg (DE); MARTENS-LOBENHOFFER, Jens, 22339 Hamburg (DE); SCHMIDT, Harry, 06120 Lieskau (DE); WOLF, Gerald, 39126 Magdeburg (DE)
(74) Vertreter: Schrell, Andreas
(86) Internationale Anmeldenummer: PCT/DE2008/001055
(87) Internationale Veröffentlichungsnummer: WO 2009/012741

(56) Entgegenhaltungen:
- WO-A-2005/070878

## Beschreibung

Die Erfindung betrifft ein Verfahren zur Synthese von A-Ring-aromatisierten Acetyl-Minocyclinen.

Minocyclin (siehe FormelIII) ist ein semisynthetisches Breitband-Antibiotikum aus der Gruppe der Tetracycline, welches seit vielen Jahren in der klinischen Praxis zur Behandlung von infektiösen Erkrankungen der Atemwege, des Urogenital- und Gastrointestinaltraktes, bei verschiedenen Hauterkrankungen, wie Akne vulgaris, Rosacea, sowie bei Trachom, Clamydien-Conjunctivitis, Borreliose u.a. eingesetzt wird.

Minocyclin hemmt die Proteinbiosynthese durch Bindung an die ribosomale 30S-Untereinheit, wodurch der Zugang von Aminoacyl-t-RNS zum RNS-Ribosomen-Komplex und damit die Verlängerung der Peptidkette verhindert wird.

Neben der bereits bekannten antibiotischen Wirkung von Minocyclin rückte kürzlich ein anderer biologischer Effekt der Substanz in den Blickpunkt der Forschung. Erste Untersuchungen zeigen, dass Minocyclin offenbar einen schützenden Effekt bei verschiedenen Entzündungsprozessen und neurodegenerativen Erkrankungen hat (Yong, V.W., Wells, J., Giuliani, F., Casha, S., Power, C., and Metz, L.M. (2004). The promise of minocycline in neurology. Lancet Neurol 3, 744-751).
So wird Minocyclin gegenwärtig zur klinischen Behandlung fortschreitender Entzündungsprozesse, wie z.B. inflammatorischer rheumatoider Arthritis vorgeschlagen (Furst, D.E. (1998). Update on clinical trials in the rheumatic diseases. Curr Opin Rheumatol 10, 123-128). Entzündungsprozesse spielen eine große Rolle in der Pathogenese von neurodegenerativen Erkrankungen, wie der Alzheimer-Krankheit, Parkinson-Krankheit, Multipler Sklerose sowie bei posttraumatischen Hirn- und Rückenmarksverletzungen.

Die Schrift DE 38 81 024 T2 offenbart ein Verfahren zur Herstellung von Tetracyclinderivaten, wie bspw. Minocyclin, dass mehrstufig ist, unter dem Einsatz von Katalysatoren auf einem Träger unter Verwendung von organischem Lösungsmittel und Druck abläuft, wobei lediglich die Enthalogenierung und Hydrierung in einem Schritt durchgeführt werden.
Der Nachteil dieses Herstellungsverfahrens ist, dass es aufwendig und damit sehr kostenintensiv ist. Darüber hinaus werden bei dem Verfahren u.a. selenhaltige Legierungen als Katalysator eingesetzt.

Die Schrift WO 2005/070878 offenbart A-Ring-aromatisierte Tetracyclinderivate und ein Verfahren zu deren Herstellung.
Die Herstellung von A-Ring-aromatisierten Tetracyclinen, wie sie in dieser Offenlegung beschrieben ist, gelingt prinzipiell, setzt jedoch mehrere Reaktionsstufen und eine aufwendige Reinigung der Reaktionsprodukte durch präparative HPLC voraus, welche für eine industrielle und somit rentable Gewinnung der Wirkstoffe, z.B. im Gramm- oder Kilogramm-Maßstab, ungeeignet ist.

Aufgrund der gefundenen biologischen und pharmakologischen Wirkungen ist die Verwendung von Minocyclin zur Behandlung neurodegenerativer Erkrankungen sehr interessant. Die Untersuchung von Minocyclin als Leitstruktur stellt dabei einen attraktiven Ansatz bei der Suche nach neuroprotektiven Wirkstoffen dar.

Eine Optimierung und Verbesserung der Minocyclin-Leitstruktur ist dabei in zwei Richtungen möglich:
1. Verbesserung der Pharmakokinetik, z.B. durch Realisierung eines 'Prodrug'-Konzeptes.
2. Aufhebung der antibiotischen Aktivität, wenn diese für die Wirkung als neuroprotektive Substanz nicht relevant ist, um einen Selektionsdruck auf Mikroorganismen in Richtung einer Resistenzentwicklung auszuschließen.

Das 'Prodrug'-Konzept ist dem Fachmann als Methode bekannt, um hydrophile Wirkstoffmoleküle chemisch so zu verändern, dass sich zum einen ihre Lipophilie und damit die Aufnahmefähigkeit durch Membranen erhöht, zum anderen die eigentlichen Wirkstoffinoleküle erst aus einer Vorstufe ('Prodrug') in den Zellen gebildet werden. Hierbei dient die Vorstufe ('Prodrug') u.a. als Transporter ('Carrier').

Eine 'Prodrug' ist per Definition ein Stoff bzw. ein Medikament, das ohne Verstoffwechselung pharmakologisch nicht oder wenig wirksam ist und erst durch die Verstoffwechselung im Körper in einen aktiven Wirkstoff überführt wird. 'Prodrugs' sind in denjenigen Fällen von strategischer Bedeutung, in denen der eigentliche Wirkstoff nur geringfügig und wenig selektiv den Wirkort erreicht.
Das 'Prodrug'-Konzept zielt dabei auf eine Verbesserung der pharmakokinetischen Eigenschaften der Wirkstoffmoleküle ab, um z.B. deren Resorbierbarkeit/Bioverfügbarkeit zu verbessern oder bei einem Psychopharmakon bzw. Neuroprotektivum die Blut-Hirn-Schranken-Gängigkeit zu ermöglichen.

Hydrophile Wirkstoffmoleküle, wie Minocyclin besitzen aufgrund von freien Hydroxyl(OH)- bzw. Amino(NH₂)-Gruppen oftmals zwar eine gute Wasserlöslichkeit, was ihre pharmazeutische Formulierbarkeit begünstigt, sind aber relativ schlecht membrangängig.
Eine Möglichkeit der Senkung ihrer Polarität ist die Einführung von apolaren Schutzgruppen. Als apolare Schutzgruppe (engl. 'prodrug moiety') hat sich die Acetyl-Gruppe (CH₃CO) bewährt, die nach Abspaltung in den Zellen freie Essigsäure bildet, welche einen natürlichen Metaboliten des Zellstoffwechsels darstellt. Sowohl OH- als auch NH₂-Gruppen lassen sich durch Acetyl-Gruppen maskieren. Die Polarität der acetylierten Moleküle ist ähnlich der biologischer Membranen, wobei eine stärkere Wechselwirkung mit Lipiden und somit erhöhte Diffusion in das Gewebe erreicht wird ('Carrier'-Effekt). Nach Aufnahme der acetylierten 'Prodrug' in die Zellen werden die Acetyl-Gruppen durch unspezifische Esterasen abgespalten, wodurch das eigentliche Wirkstoffmolekül endogen freigesetzt wird und hier in Aktion treten kann.

Die 'Prodrug' stellt außerdem eine Retardform des Wirkstoffmoleküls dar, die eine verzögerte Freisetzung und ein günstigeres pharmakologisches Verhalten impliziert.
In der Literatur sind bisher nur wenige vom Minocyclin abgeleitete neuroprotektive Wirkstoffmoleküle beschrieben, die zwar den Naturstoff zum Vorbild haben, mit denen jedoch bisher kein 'Prodrug'-Konzept verfolgt wird [Wang, R., Du, Y., and Liu, Zhou, Z., Wang, H., Wang, X., (J. (2004). Synthesis and neuroprotective activity of novel C4, C7 derivates in tetracycline series. J Chin Pharm Sei 13, 217-220].

Die Aufgabe der vorliegenden Erfindung ist es, die Nachteile des Standes der Technik zu vermeiden, in dem ein aufwandgeringes Verfahren zur Herstellung von A-Ring-aromatisierten Acetyl-Minocyclinen der Formel I bereitgestellt wird, das auch in industriellem Maßstab durchführbar ist.

Die vorliegende Erfindung löst dieses Problem durch die Bereitstellung der Lehre der unabhängigen Verfahrens-, Produkt- und Verwendungsansprüche.

Hierzu wird erfindungsgemäß vorgeschlagen, eine Ein- oder Mehrfach-Acetylierung der Leitstruktur unter gleichzeitiger Wasserabspaltung und Aromatisierung des A-Ringes in einem Schritt durchzuführen, um zu einem neuroprotektiven Wirkstoff zu gelangen.

Dabei zeigt sich überraschenderweise, dass durch eine Einstufen-Reaktion von Minocyclinhydrochlorid mit Essigsäureanhydrid (Acetanhydrid) in Gegenwart eines organischen Protonenfängers A-Ring-aromatisierte ein- oder mehrfachacetylierte Minocycline der Formel I (wobei R¹ bis R⁵ = Acetyl und / oder H sind) durch Acetylierung von Minocyclinhydrochlorid, die präparative chromatographische Aufreinigung bzw. Trennung der Reaktionsprodukte und die anschließende weiterführende Reinigung durch Umkristallisation der entsprechenden Produkte aus einem Ethylacetat / Petroleumbenzin-Gemisch erhalten werden.

Solche A-Ring-aromatisierten Acetyl-Minocycline der Formel I (mit R¹ bis R⁵ = Acetyl und/oder H) setzen nach Verstoffwechselung im Organismus A-Ring-aromatisiertes Minocyclin der Formel I (mit R¹ bis R⁵ = H) frei, welches eine zell- bzw. neuroprotektive, jedoch keine antibiotische Wirkung mehr aufweist.

Die Herstellung von ein- oder mehrfachacetylierten A-Ring-aromatisierten Minocyclinen der Formel I (wobei R¹ bis R⁵ = Acetyl und / oder H sind) erfolgt durch die Reaktion von Minocyclinhydrochlorid mit Acetanhydrid in Gegenwart eines organischen Protonenfängers. Dabei wird vorzugsweise ein äquimolarer Überschuss an Acetanhydrid bzw. organischem Protonenfänger verwendet, wobei diese gleichzeitig Lösungsmittel für das Minocyclinhydrochlorid sind.
Genauso können aber auch äquimolare Mengen der Edukte, entsprechend der Anzahl einzuführender Acetyl-Gruppen eingesetzt werden, wobei verminderte Mengen an Acetanhydrid und Protonenfänger gegebenenfalls durch ein inertes Lösungsmittel zu ersetzen sind. Geeignete inerte Lösungsmittel für die Edukte sind beispielsweise Chloroform, Methylenchlorid, Nitromethan, Acetonitril, Aceton, Sulfolan, Dimethylformamid oder Dimethylsulfoxid.

Als organischer Protonenfänger wird bevorzugt Pyridin verwendet, wobei man die Reaktion in einem Temperaturbereich von 4 bis 100 °C, besonders bevorzugt bei 75 °C bzw. unterhalb der Siedetemperatur des Reaktionsgemisches ablaufen lässt.
Anstelle von Pyridin können auch andere Protonenfänger wie beispielsweise primäre, sekundäre oder tertiäre Amine bzw. Carbonsäureamide verwendet werden.

Die Umsetzung wird üblicherweise bei Normaldruck unter Rühren der Reaktionsmischung durchgeführt. Dabei verwendet man eine Glas-Reaktionsappartur mit Rückflusskühler, wobei entstehende Lösungsmitteldämpfe kondensiert und kontinuierlich in die Reaktionsmischung zurückgeführt werden.

Das erfindungsgemäße Verfahren kann jedoch auch unter verminderten oder erhöhten Druck durchgeführt werden. Die Anwendung von erhöhtem Druck ist insbesondere dann angezeigt, wenn man die Reaktion bei einer Temperatur durchführen will, bei der das Lösungsmittel unter Normaldruck siedet.

Überraschenderweise können mittels des erfindungsgemäßen Verfahrens in einem Reaktionsschritt die beiden neuartigen Substanzen Pentaacetylcyclin (A-Ring-aromatisiertes Pentaacetyl-Minocyclin) und Tetraacetylcyclin (A-Ring-aromatisiertes Tetraacetyl-Minocyclin) gewonnen werden, welche bisher nicht publiziert sind.

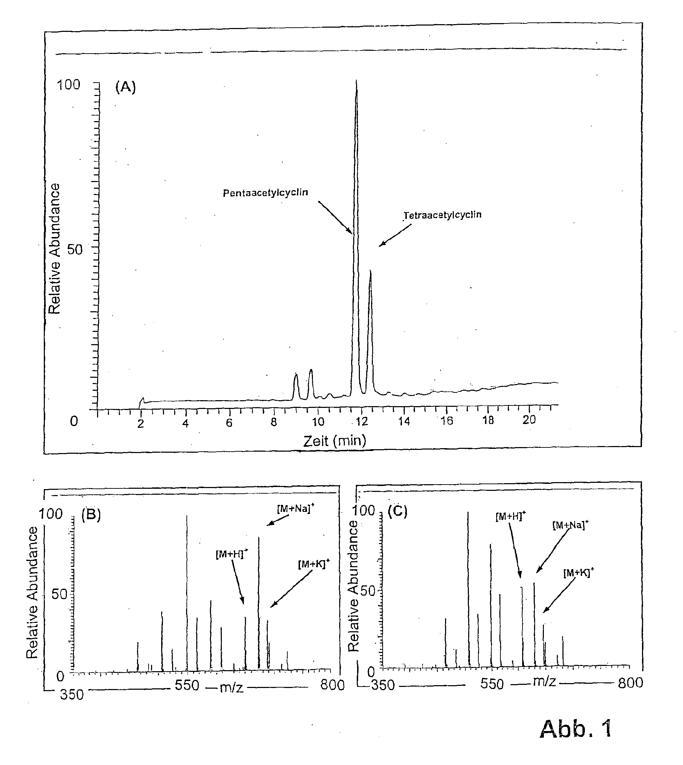

Abbildung 1 zeigt beispielhaft das LC(HPLC)-Chromatogramm und die korrespondierenden Massenspektren der zwei Hauptkomponenten eines Reaktionsgemisches nach Einsatz von überschüssigem Acetanhydrid. Es ist die hohe Selektivität der Reaktion für das pentaacetylierte Produkt (Formel II und Abbildung 1B) erkennbar. Durch Optimierung der Reaktionsbedingungen kann die Selektivität für das entsprechende Zielprodukt noch weiter erhöht werden, wodurch sich nachfolgend beschriebene Reinigungsschritte vereinfachen bzw. erübrigen.

In dem hier beschriebenen erfindungsgemäßen Verfahren wird nach Ablauf der Reaktionszeit eine chromatographische Trennung bzw. Reinigung der Produkte mit Hilfe der VLC (Vakuum-Flüssigkeitschromatographie) durchgeführt.
Die VLC-Methode kann, je nach technischer Ausführung, auch im industriellen Maßstab durchgeführt werden. Dazu wird das Reaktionsgemisch vorzugsweise auf ein mit Petroleumbenzin (Siedebereich: 40 - 60 °C) oder einem anderen Kohlenwasserstoff, wie *n*-Pentan, *n*-Hexan, Cyclohexan oder Isobutan vorkonditioniertes Trägermaterial gegeben, welches sich in einem Büchner-Trichter mit Frittenboden, einer Chromatographie-Säule oder einem Hängegefäß mit Siebboden befindet. Durch Vakuum-Zug (VLC) oder mit Hilfe von Überdruck wird der Eluent nun durch das Trägermaterial (stationäre Phase) geleitet.
Die Elution der Zielverbindungen erfolgt anschließend durch Aufgabe eines Lösungsmittel-Gradientengemisches, bestehend aus einem Kohlenwasserstoff und einem Carbonsäureester, wobei man die Polarität des Gemisches durch höhere Anteile des Carbonsäureesters während der Elution steigert.

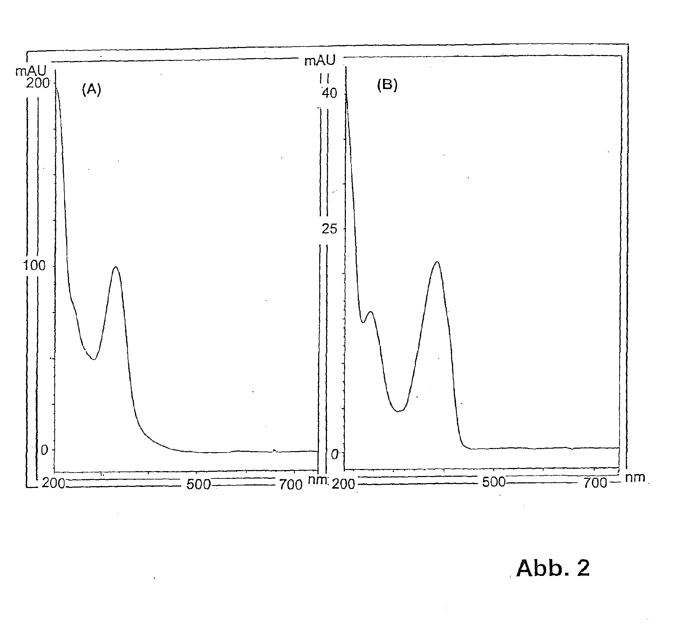

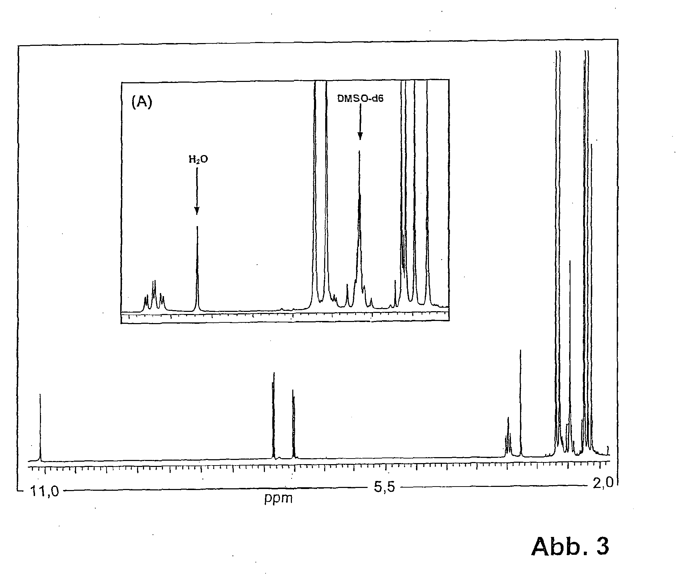

In dem hier vorgestellten erfindungsgemäßen Verfahren wird zur Elution der Zielverbindungen besonders bevorzugt ein Gemisch aus Petroleumbenzin und Ethylacetat verwendet. Es ist jedoch auch möglich, dass anstelle dessen Eluenten-Gemische von Kohlenwasserstoffen mit anderen Carbonsäureestern, wie Methylformiat, *n*-Butylacetat oder Dimethylcarbonat, verwendet werden können.
Als Trägermaterial (stationäre Phase) werden Kieselgel (z.B. Kieselgel 60), Aluminiumoxid, 'reversed phase'-Kieselgel oder Sephadex verwendet.
Nach Abtrennung entsprechender Fraktionen, welche die angereicherten Zielverbindungen enthalten, wird der Eluent unter vermindertem Druck durch Verwendung eines Rotationsverdampfers abdestilliert und der Rückstand aus einem der vorstehend genannten Lösungsmittel-Gemische umkristallisiert.
Zur Umkristallisation wird im erfindungsgemäßen Verfahren die entsprechende Zielverbindung zunächst unter leichtem Erwärmen in Ethylacetat gelöst und dann durch Zusatz von Petroleumbenzin, somit durch Verminderung des Löslichkeitsproduktes, eine Kristallisation eingeleitet.
Nach vollständiger Kristallisation, wobei man die Mischung für einige Stunden auf 4 °C kühlt, filtriert man die Zielverbindung mit Hilfe einer Filternutsche ab und entfernt Lösungsmittelreste durch Trocknung im Vakuum.
Die hier im Folgenden beispielhaft aufgeführten, vermittels des erfindungsgemäßen Verfahrens hergestellten neuartigen Substanzen Pentaacetylcyclin (A-Ring-aromatisiertes Pentaacetyl-Minocyclin der Formel II) und Tetraacetylcyclin (A-Ring-aromatisiertes Tetraacetyl-Minocyclin der Formel IV) wurden durch Massenspektroskopie (LC/MS, HR/MS), UV/VIS-Spektroskopie sowie durch ¹H- und ¹³C-NMR charakterisiert.
So zeigt Abbildung 2 beispielhaft die UV/VIS-Spektren beider vorstehend genannter Substanzen und Abbildung 3 das ¹H-NMR von Tetraacetylcyclin der Formel IV.
Die Prüfung der zellschützenden Eigenschaften der durch das erfindungsgemäße Verfahren dargestellten Substanzen erfolgte, beispielhaft an rein dargestelltem Pentaacetylcyclin der Formel II, mit Hilfe eines Astrozytenschädigungsmodells. Dieses Modell dient der Untersuchung durch oxidativen Stress vermittelter neurodegenerativer Erkrankungen bzw. zur Testung von zellschützenden Substanzen, mit denen eine solche Schädigung verhindert bzw. verringert werden kann. In dem beispielhaft verwendeten Modell werden Astrozyten mit Hilfe von Wasserstoffperoxid (H₂O₂) geschädigt und anschließend die Funktionalität und Morphologie der Zellen und Mitochondrien mikroskopisch beurteilt. Eine Überproduktion von H₂O₂, als reaktive Sauerstoffspezies (ROS), gilt im ZNS u.a. als Auslöser vieler neurodegenerativer Prozesse und Erkrankungen, wie Schlaganfall, Alzheimer-Krankheit, Parkinson-Krankheit, post-traumatischen Hirn- und Spinalverletzungen. Erstaunlicherweise vermochte Pentaacetylcyclin der Formel II gegenüber der Vergleichssubstanz Minocyclinhydrochlorid bereits in viel niedrigerer Dosierung die Schädigung der Mitochondrien deutlich zu vermindern (Beispiel 3 und Abbildung 4). Dieser Befund ist überraschend und zeigt die herausragenden Wirkeigenschaften der beispielhaft getesteten Substanz.

Die Überprüfung der antibiotischen Aktivität des durch das erfindungsgemäße Verfahren gewonnenen Pentaacetylcyclins erfolgte unter Verwendung eines *E*. *coli*-Stammes (Beispiel 4). Pentaacetylcyclin weist im Vergleich zum Minocyclinhydrochlorid keine antibiotische Wirkung mehr auf, womit überraschenderweise ein weiterer angestrebter biologischer Effekt erreicht wurde.

Die Erfindung wird nachstehend an Hand der folgenden Ausführungsbeispiele und Abbildungen näher erläutert.
- Abb.l:: Chromatogramm einer LC/MS-Analyse des erfindungsgemäßen Reaktionsgemisches vor der Aufreinigung, wobei
(A) -Analytisches LC-Chromatogramm des Reaktionsgemisches,
(B) -Massenspektrum des Pentaacetylcyclins (Analyse des Peak bei 11,69 min) und
(C) -Massenspektrum des Tetraacetylcyclins (Analyse des Peak bei 12,37 min) bedeuten,
- Abb. 2:: UV/VIS-Spektren von Pentaacetylcyclin (A) und Tetraacetylcyclin (B), hergestellt gemäß dem erfindungsgemäßen Verfahren, sowie
- Abb.3:: ¹H-NMR-Spektrum von Tetraacetylcyclin (in DMSO-d6) hergestellt gemäß dem erfindungsgemäßen Verfahren, wobei (A) eine vergrößerte Darstellung des Bereich von 2.0 - 3.6 ppm darstellt.

### Ausführungsbeispiel 1

### Synthese von Pentaacetylcyclin der Formel II

Minocyclinhydrochlorid (0,60 g; 1,215 mmol) wurde unter Eiskühlung (4 °C) in 12 ml Pyridin (11,73 g; 148,37 mmol) gelöst und anschließend unter Rühren 12 ml Acetanhydrid (12.98 g, 127.18 mmol) hinzugefügt. Dann wurde die Mischung 30 Minuten bei 4 °C, 2 Stunden bei Raumtemperatur und 30 Minuten bei 75 °C nachgerührt. Die Reinigung des Reaktionsproduktes erfolgte durch Vakuumflüssigkeits-Chromatographie (VLC). Dazu wurde Kieselgel 60 (75 g) in einer Glasfilternutsche unter Vakuumzug verdichtet und mit Petroleumbenzin (Siedebereich: 40 - 60 °C) vorkonditioniert. Das Reaktionsgemisch wurde auf die so vorbereitete Chromatographiesäule geladen und mit einem Petroleumbenzin / Ethylacetat-Gradienten (4/1, vol/vol bis 100 % EtOAc) eluiert. Fraktionen, die das Reaktionsprodukt enthielten, wurden vereinigt. Nach Abdestillieren des Lösungsmittels am Rotationsverdampfer wurde ein Rückstand (518,5 mg) erhalten, der aus einem Ethylacetat / Petroleumbenzin-Gemisch umkristallisiert wurde und schließlich das fast reine Pentaacetylcyclin als hellgelben Feststoff lieferte. Die Roh-Ausbeute betrug 237,5 mg. Zur Endreinigung wurde das Produkt einer weiteren VLC unterworfen. Die Reinausbeute betrug 181,9 mg (23 % d. Theorie). Die analytische Kontrolle der jeweiligen Fraktionen erfolgte über Dünnschichtchromatographie (DC mit Fluoreszenzindikator, Polygram SIL G/UV₂₅₄, Firma Macherey & Nagel) mit Kieselgel 60 als stationäre Phase und Petroleumbenzin/Ethylacetat (1/3, vol/vol) als mobile Phase. Für Zellkulturexperimente bzw. zur Testung der antibiotischen Wirkung wurden Stammlösungen der Substanz in DMSO hergestellt, mit Hilfe von Nalgene-Nylonfiltern (0,22 µm Porengröße) steril filtriert und bis zur Verwendung bei -20 °C gelagert.

### Analytische Daten Pentaacetylcyclin (A-Ring aromatisiertes Pentaacetyl-Minocyclin):

- R_{f} (DC) = 0,50
- UV-VIS (λₘₐₓ in MeOH): 330 nm (log ε 4,27)
- LC/MS (positive ion mode): 723 [M + C₃H₆O₂]⁺, 688 [M + K]⁺, 672 [M + Na]⁺, 650 [M + H]⁺, 608 [650 - Acetyl + H]⁺, 590 [608 - H₂O], 565 [608 - Acetyl]⁺, 548 [590 - Acetyl + H]⁺, 506 [548 - Acetyl + H]⁺
- HR-ESI-MS (negative ion mode): (M - H⁻) gef. 648,2198825, C₃₃H₃₄O₁₁N₃, dev. 2,5 ppm
- HR-ESI-MS: (M - Acetyl) gef. 606,2093178 (M - Acetyl), C₃₁H₃₂O₁₀N₃, dev. 1,2 ppm
- HR-ESI-MS (positive ion mode): (M+Na⁺) gef. 672,21638, C₃₃H₃₅O₁₁N₃Na, dev. 2,4 ppm
- ¹H-NMR (DMSO-d6): δ (ppm) 2,16 (s, CH₃); 2,23 (s, CH₃); 2,27 (s, CH₃); 2,28 (s, 2 CH₃); 2,56 (m, CH); 2,66 (s, N(CH₃)₂); 2,73 (s, N(CH₃)₂); 2,76 (m, CH₂, überlagert von N(CH₃)₂-Signal); 3,48 (m, CH₂); 7,00 (d, *J* = 8,6 Hz, aromat. CH); 7,29 (d, *J* = 8,2 Hz, aromat. CH); 11,08 (br s, NH)
- ¹³C-NMR (DMSO-d6): Signale, Auswahl δ (ppm) 13,9; 20,2; 20,6; 24,6; 31,7; 42,4; 43,9; 59,5; 122,0; 124,5; 139,9; 148,5; 161,7; 167,2; 167,8; 168,1; 168,9; 169,8

### Ausführungsbeispiel 2

### Synthese von A-Ring-aromatisiertem Tetraacetyl-Minocyclin der Formel IV

Tetraacetylcyclin wurde als Nebenprodukt aus dem, im Ausführungsbeispiel beschriebenen Reaktionsgemisch chromatographisch isoliert und anschließend aus der entsprechenden Fraktion, nach Abdestillieren des Lösungsmittels und Umkristallisation aus einem Petroleumbenzin/Ethylacetat-Gemisch, rein gewonnen. Aufgrund des bathochromen Shifts der UV-Bande gegenüber der des Pentaacetylcyclins der Formel II ist die Enol-Form, entsprechend Formel IV, am wahrscheinlichsten (Abbildung 2). Die anderen (nicht enolischen) OH- bzw. die NH₂-Gruppen im Minocyclin weisen eine höhere Basizität auf und werden deshalb bevorzugt acetyliert. Das ¹H-NMR-Spektrum (Abbildung 3) unterstützt den Strukturbeweis.

### Analytische Daten Tetraacetylcyclin (A-Ring aromatisiertes Tetraacetyl-Minocyclin):

- R_{f} (DC) = 0,57
- UV-VIS (λmax): 252,381 (log s 4,27)
- LC/MS (positive ion mode): 681 [M + C₃H₆O₂]⁺, 646 [M + K]⁺, 630 [M + Na]⁺, 608 [M + H]⁺, 566 [608 - Acetyl + H]⁺, 548 [566 - H₂O]⁺, 524 [566 - Acetyl + H]⁺, 506 [524 - H₂O]⁺
- ¹H-NMR (DMSO-d6): δ (ppm) 2,16 (s, CH₃); 2,22 (s, CH₃); 2,27 (s, CH₃); 2,29 (s, CH₃); 2,49 (dt, *J* = 13,7 Hz, CH₂); 2,63 (m, *J* = 4,5; 13,6 Hz; CH); 2,66 (s, N(CH₃)₂); 2,72 (s, N(CH₃)₂); 3,50 (dt, *J* = 4,5; 14,9 Hz; CH); 7,04 (d, *J* = 8,6 Hz; aromat. CH); 7,37 (d, *J* = 8,6 Hz; aromat. CH); 11,10 (s, NH)

### Ausführungsbeispiel 3

### Prüfung der zell- bzw. neuroprotektiven Wirkeigenschaften am Astrozyten-Mitochondrien-Modell

Astrogliazellen, d.h. nicht-neoplastische embryonale Astrozyten-Zelllinie der Ratte [Chamaon, K., Kirches, E., Kanakis, D., Braeuninger, S., Dietzmann, K., and Mawrin, C. (2005). Regulation of the pituitary tumor transforming gene by insulin-like-growth factor-I and insulin differs between malignant and non-neoplastic astrocytes. Biochem Biophys Res Commun 331, 86-92] wurden auf mit Poly-D-Lysin beschichteten Deckgläschen am Boden von Kulturschalen für 18 Stunden bei 37 °C (5 % CO₂) kultiviert. Als Kulturmedium diente jeweils 2 ml DMEM (PAA Laboratories GmbH Pasching, Österreich) und die Einsaatdichte betrug 0,3 x 10⁶ Zellen/2 ml. Die Zellkulturen wurden zunächst mit unterschiedlichen Konzentrationen (1,0; 5,0 bzw. 25,0 µM) Minocyclinhydrochlorid bzw. Pentaacetylcyclin (Formel II) für 30 Minuten vorinkubiert. Als unbehandelte Kontrolle diente die alleinige Zugabe des Lösungsmittels DMSO (2 µl/Kulturschale) unter identischen Inkubationsbedingungen. Danach erfolgt die Zugabe von 1,0 bzw. 3,0 mM H₂O₂ (Endkonzentration) und eine weitere Inkubation für 2 Stunden. Als Vergleich dienten die entsprechend behandelten Zellen, welche nur mit 1,0 bzw. 3,0 mM H₂O₂ oder nur mit Minocyclinhydrochlorid bzw. Pentaacetylcyclin, in den angegebenen Konzentrationen kultiviert wurden. Nach Austausch des Kulturmediums gegen 2 ml auf 37 °C temperierten HEPES-Puffer (10 mM HEPES, 140 mM NaCl, 5 mM KCl, 2 mM CaCl₂, 10 mM D-Glukose, pH 7,4) fand eine weitere Inkubation der Zellen für 30 Minuten statt. Darauf folgte die Zugabe von 40 nM MitoTracker^{®} Orange CMTMRos (1 µl einer 80 µM Stammlösung der oxidierten Form, Bestell-Nr. M7510; λ_{Anregung} = 554 nm, λ_{Emission} = 576 nm; Molecular Probes Inc., Eugene, USA). Nach 30-minütiger Inkubation bei 37 °C wurden die Zellkulturen in 4% Paraformaldehyd (m/vol in HEPES-Puffer) für 30 Minuten fixiert, zur Entfernung des Fixationsmittels dreimal für 10 Minuten mit 0,1 M PBS gespült und anschließend die Deckgläschen mit den fixierten Zellen auf Objektträger in ImmuMount^{®} befestigt. Zur visuellen Untersuchung und Dokumentation kam ein Zeiss-Axiophot-Fluoreszensmikroskop mit CCD-Kamera (AxioCam MRc) zum Einsatz.
Unbehandelte Kontrollzellen stellen sich als polygonale Zellkörper mit 2-3 langgestreckten Fortsätzen dar und erscheinen bei ausreichender Zelldichte als ein zelluläres Netz. Fadenförmige Mitochondrien sind im Zytoplasma bis in die Zellfortsätze hinein verteilt. Nach 2-stündiger Inkubation mit 1 mM H₂O₂ (ohne Minocyclin bzw. Pentaacetylcyclin) sind die Mehrzahl der Zellen geschädigt und zeigen einen deutlichen Rückzug ihrer Fortsätze. Dadurch verlieren die Zellkörper ihre polygonale Form und tendieren zur Abrundung. Infolge von Fission sind die Mitochondrien stark verkürzt und werden in Richtung des Zellkerns disloziert. Mit 3 mM H₂O₂ sind diese zellschädigenden Prozesse verstärkt. Die Dichte der haftenden Zellen ist verringert, die Zellkörper sind weit mehr abgerundet und die noch stärker verkürzten Mitochondrien sind nahe dem Zellkern akkumuliert. Die Zugabe von 25 µM Minocyclinhydrochlorid zu den zuvor mit H₂O₂ geschädigten Zellen hat einen schützenden Effekt. Die Zellen erhalten weitgehend ihre polygonale Gestalt und die Mitochondrien sind weniger verkürzt. Überraschenderweise zeigte sich, dass bei Verwendung von Pentaacetylcyclin schon bei der viel niedrigeren Konzentration von 1,0 µM die H₂O₂-induzierte Schädigung der Mitochondrien deutlich verringert wird. Dieser Schutzeffekt des A-Ring-aromatisierten Pentaacetyl-Minocyclins ist eine Verbesserung gegenüber Minocyclinhydrochlorid, mit welchem erst ab 25 µM eine ähnliche Schutzwirkung beobachtet werden kann. In dem genutzten Testsystem und den hier verwendeten Konzentrationen schädigen Minocyclinhydrochlorid und Pentaacetylcyclin die Zellen nicht.

### Ausführungsbeispiel 4

### Prüfung der antibiotischen Wirkung von Pentaacetylcyclin und Minocyclinhydrochlorid im Plattendiffusionstest

Die Flüssigkultur (24 Stunden in LB-Lennox-L-Broth-Base kultiviert) einer *E*. *coli*-Suspension (150 µl) des Stammes C 600 hfc wurde auf LB-Agar (Lennox-L-Agar, Gibco) mit Hilfe eines sterilen Drigalski-Spatels gleichmäßig verteilt. Anschließend wurden sterile Filterpapierblättchen (Durchmesser 5,3 mm), die zuvor mit sterilen DMSO-Lösungen sowie unterschiedlichen Konzentrationen (100 µM bis 2,5 mM) von Minocyclinhydrochlorid bzw. Pentaacetylcyclin (Formel II) getränkt wurden, auf den Nährboden aufgelegt. Nach Inkubation der Platten bei 37 °C für 24 Stunden erfolgte die Beurteilung der antibiotischen Aktivität der Substanzen anhand der Durchmesser der Hemmhöfe, welche durch die fehlende Eintrübung durch Bakterien sichtbar werden. An Hand der unterschiedlichen Größe der Hemmhöfe ist erkennbar, dass Minocyclinhydrochlorid konzentrationsabhängig das Wachstum von *E. coli* inhibiert. Hingegen zeigt Pentaacetylcyclin selbst in der höchsten getesteten Konzentration von 2,5 mM keine Wachstumshemmung und ist demzufolge gegen den verwendeten *E. coli* Stamm nicht antibiotisch wirksam.

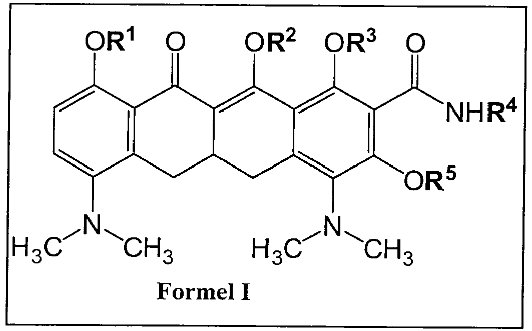

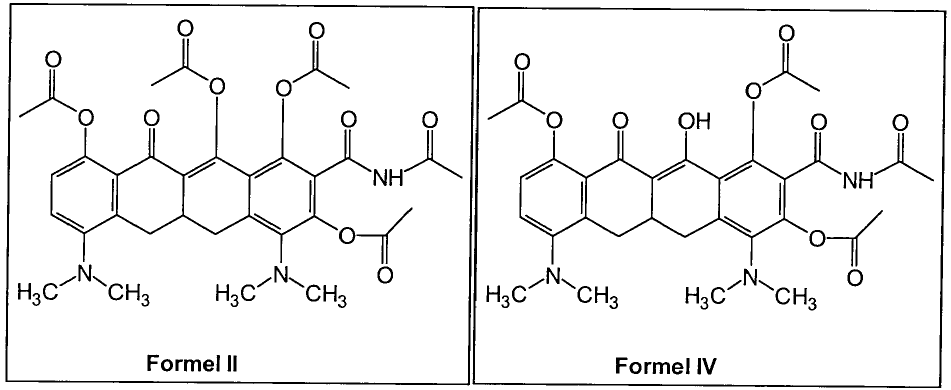

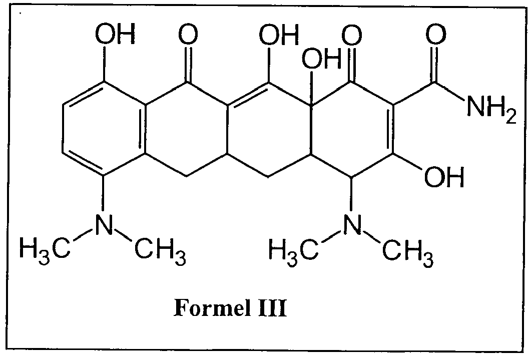

## Patentansprüche

1. Verfahren zur Herstellung von A-Ring-aromatisierten Acetyl-Minocyclinen 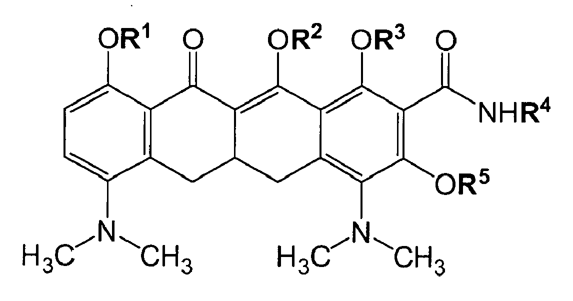 wobei R¹ bis R⁵ = Acetyl und/oder H sind, **dadurch gekennzeichnet, dass** Minocyclinhydrochlorid mit Acetanhydrid in Gegenwart eines Protonenfängers umgesetzt wird, wobei eine Ein- oder Mehrfach-Acetylierung der Leitstruktur unter gleichzeitiger Wasserabspaltung und Aromatisierung des A-Rings erfolgt, das Reaktionsprodukt einer chromatographischen Aufreinigung unter Verwendung eines Trägermaterials und eines Eluenten unterworfen, der Eluent abdestilliert und das Produkt anschließend durch Umkristallisation gereinigt wird, wobei mindestens ein R = Acetyl ist.

2. Verfahren gemäß Anspruch 1, **dadurch gekennzeichnet, dass** als Protonenfänger eine organische Base verwendet wird.

3. Verfahren gemäß den Ansprüchen 1 und 2, **dadurch gekennzeichnet, dass** die Base ein primäres, sekundäres oder tertiäres Amin ist.

4. Verfahren gemäß Anspruch 1 oder 2, **dadurch gekennzeichnet, dass** der Protonenfänger bevorzugt Pyridin ist.

5. Verfahren gemäß Anspruch 1 oder 2, **dadurch gekennzeichnet, dass** das Acetanhydrid und der Protonenfänger im Überschuss oder äquimolar, entsprechend der Anzahl der einzuführenden Acetylgruppen, eingesetzt werden.

6. Verfahren nach einem oder mehrerer der voran stehenden Ansprüche, **dadurch gekennzeichnet, dass** die Umsetzung der Edukte in einem inerten Lösungsmittel erfolgt.

7. Verfahren gemäß Anspruch 6, **dadurch gekennzeichnet, dass** das Lösungsmittel Chloroform, Methylenchlorid, Nitromethan, Acetonitril, Aceton, Sulfolan, Dimethylformamid oder Dimethylsulfoxid ist.

8. Verfahren nach einem oder mehrerer der voran stehenden Ansprüche, **dadurch gekennzeichnet, dass** die Umsetzung bei einer Temperatur von 4 bis 100° C, bei Normal- oder Überdruck durchgeführt wird.

9. Verfahren nach einem oder mehrerer der voran stehenden Ansprüche, **dadurch gekennzeichnet, dass** Pentaacetylcyclin (A-Ring-aromatisiertes Pentaacetyl-Minocyclin) der Formel II 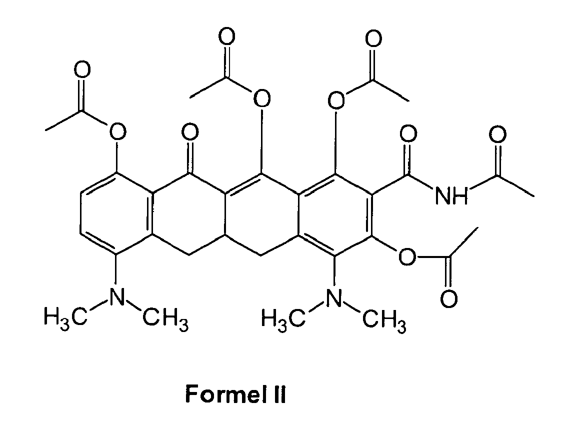 als Hauptprodukt synthetisiert wird.

10. Verfahren nach einem oder mehrerer der voran stehenden Ansprüche, **dadurch gekennzeichnet, dass** je nach Reaktionsführung andere A-Ring-aromatisierte, ein- oder mehrfachacetylierte Minocycline der Formel I (mit R¹ bis R⁵ = Acetyl und/oder H, wobei mindestens ein R =Acetyl) synthetisiert werden, wobei die Anzahl der Acetylgruppen im Molekül kleiner oder gleich 5 ist.

11. Verfahren nach einem oder mehrerer der voran stehenden Ansprüche, **dadurch gekennzeichnet, dass** als Nebenprodukt besonders bevorzugt das Tetraacetylcyclin (A-Ring-aromatisiertes Tetraacetyl-Minocyclin) der Formel IV 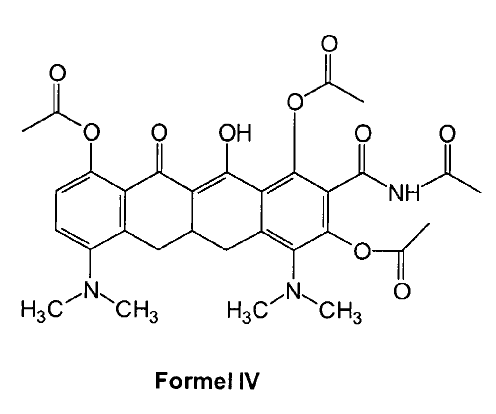 synthetisiert wird.

12. Verfahren nach einem oder mehrerer der voran stehenden Ansprüche, **dadurch gekennzeichnet, dass** die chromatographische Reinigung der Reaktionsprodukte an Kieselgel 60 (Silicagel) oder einem anderen Trägermaterial wie Aluminiumoxid, 'reversed-phase'-Silicagel oder Sephadex durchgeführt wird.

13. Verfahren nach einem oder mehrerer der voran stehenden Ansprüche, **dadurch gekennzeichnet, dass** die chromatographische Aufreinigung im Festbett, in einer Chromatographiesäule, in einem Büchner-Trichter mit Glasfritten-Einsatz oder einem Hängegefäß mit Siebboden-Einlage erfolgt.

14. Verfahren nach einem oder mehrerer der voran stehenden Ansprüche, **dadurch gekennzeichnet, dass** zur Elution über das Trägermaterial und zur Umkristallisation der Reaktionsprodukte besonders bevorzugt ein Gemisch aus Ethylacetat und Petroleumbenzin verwendet wird.

15. Verfahren nach einem oder mehrerer der voran stehenden Ansprüche, **dadurch gekennzeichnet, dass** an Stelle von Ethylacetat und Petroleumbenzin auch Methylformiat, *n*-Butylacetat, Dimethylcarbonat bzw. andere Kohlenwasserstoffe wie *n*-Pentan, *n*-Hexan, Cyclohexan oder Isobutan eingesetzt werden.

16. A-Ring-aromatisiertes Pentaacetylminocyclin der Formel II

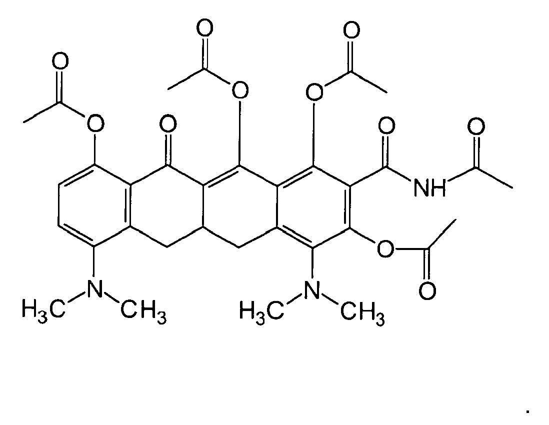

17. A-Ring-aromatisiertes Tetraacetylminocyclin der Formel IV

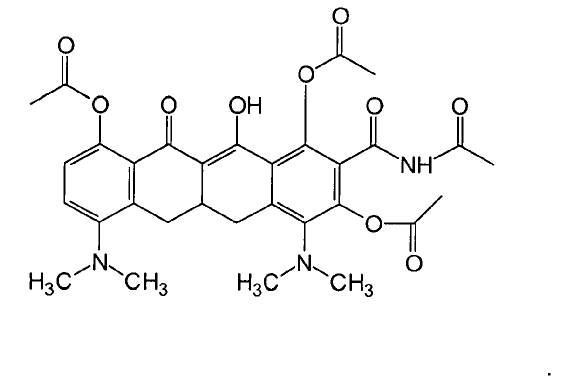

18. Verwendung des Wirkstoffes gemäß einem der Ansprüche 16 oder 17 zur Herstellung eines Medikamentes, welches zur Behandlung von durch oxidativen Stress und/oder mitochondriale Schädigungen vermittelter neurodegenerativer Erkrankungen eingesetzt werden kann.

## Claims

1. Method for the production of A-ring aromatized acetyl minocyclines 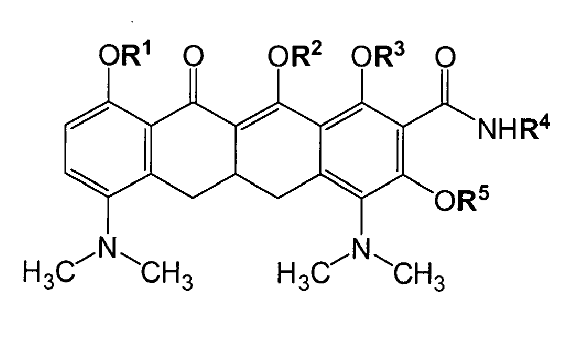 wherein R¹ to R⁵ = acetyl and/or H, **characterized in that** minocycline hydrochloride is reacted with acetanhydride in the presence of a proton catcher, wherein a single or multiple acetylation of the lead structure and simultaneous dehydration and aromatization of the A-ring occurs, the reaction product is chromatographically purified by using a carrier material and an eluent, the eluent is distilled off and afterwards the product is purified by recrystallization wherein at least one R = acetyl.

2. Method according to claim 1, **characterized in that** an organic base is used as proton catcher.

3. Method according to claims 1 and 2, **characterized in that** the base is a primary, secondary or tertiary amine.

4. Method according to claim 1 or 2, **characterized in that** pyridine is preferably used as the proton catcher.

5. Method according to claim 1 or 2, **characterized in that** the acetanhydride and the proton catcher are used in excess amounts or equimolar according to the number of the acetyl groups to be introduced.

6. Method according to one or several of the previous claims, **characterized in that** the reaction of the starting materials is performed in an inert solvent.

7. Method according to claim 6, **characterized in that** the solvent is chloroform, methylene chloride, nitromethane, acetonitrile, acetone, sulfolane, dimethylformamide or dimethylsulphoxide.

8. Method according to one or several of the previous claims, **characterized in that** the reaction is performed at a temperature ranging from 4 to 100°C, at normal pressure or overpressure.

9. Method according to one or several of the previous claims, **characterized in that** pentaacetyl cyclin (A-ring aromatized pentaacetyl minocycline) of Formula II 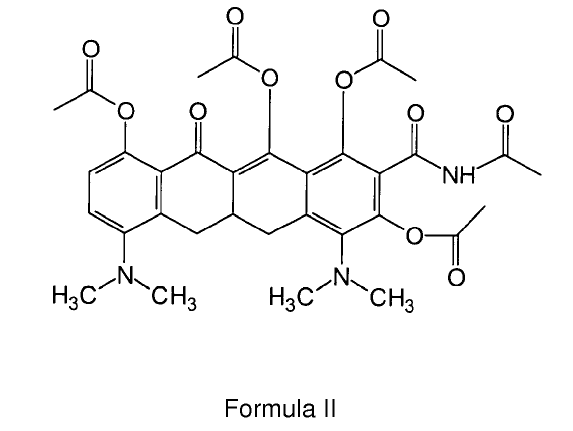 is synthesized as the main product.

10. Method according to one or several of the previous claims, **characterized in that** other A-ring aromatized, single- or multiple-acetylated minocyclines of Formula I (wherein R¹ to R⁵ = acetyl and/or H, wherein at least one R = acetyl), depending on the reaction control, are synthesized and the number of the acetyl groups in the molecule is 5 or less.

11. Method according to one or several of the previous claims, **characterized in that** in particular tetraacetyl cyclin (A-ring aromatized tetraacetyl minocycline) of Formula IV 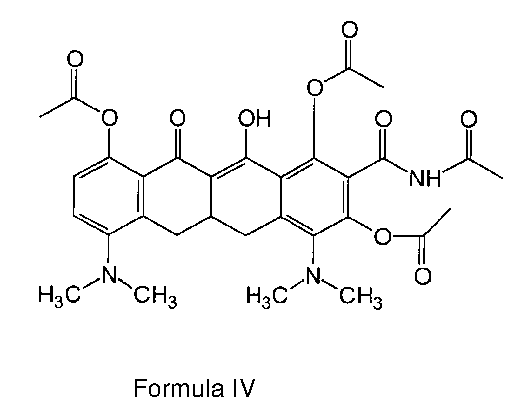 is preferably synthesized as a by-product.

12. Method according to one or several of the previous claims, **characterized in that** the chromatographic purifying of the reaction products is performed at silica gel 60 (Silicagel) or another carrier material, such as aluminum oxide, 'reversed-phase' silica gel or Sephadex.

13. Method according to one or several of the previous claims, **characterized in that** the chromatographic purifying is performed in a packed bed, in a chromatographic column, in a Büchner funnel provided with a glass fritted insert or in a suspended vessel with sieve bottom insert.

14. Method according to one or several of the previous claims, **characterized in that** in particular a mixture of ethyl acetate and petroleum ether is preferably used for the elution via the carrier material and for the recrystallization of the reaction products.

15. Method according to one or several of the previous claims, **characterized in that** methyl formate, *n*-butyl acetate or dimethyl carbonate or another hydrocarbon, such as *n*-pentan, *n*-hexan, cyclohexan or isobutan, can also be used instead of ethyl acetate and petroleum ether.

16. A-ring aromatized pentaacetyl minocycline of Formula II

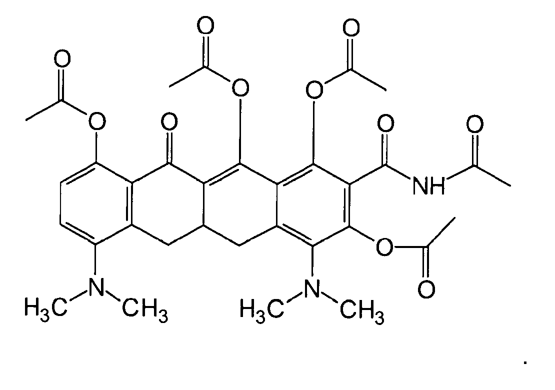

17. A-ring aromatized tetraacetyl minocycline of Formula IV

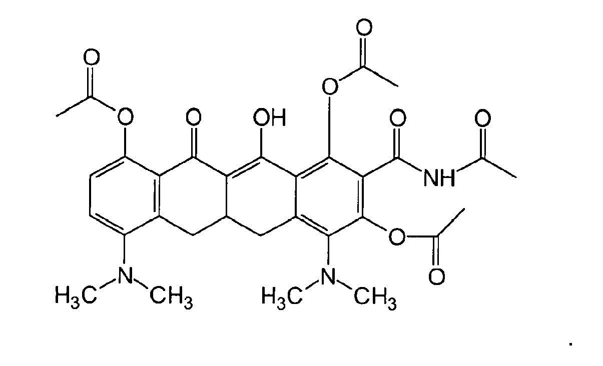

18. Use of the agent according to one of claims 16 or 17 for the production of a medicine that can be used for the treatment of neurodegenerative diseases caused by oxidative stress and/or mitochondrial damages.

## Revendications

1. Procédé de fabrication d'acétyl-minocyclines à cycle A aromatisé 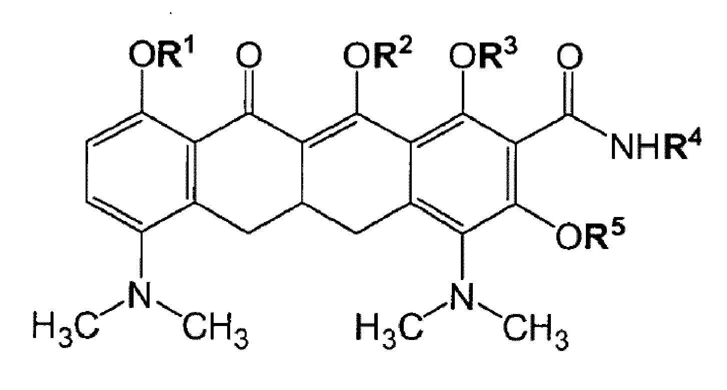 dans laquelle R¹ à R⁵ sont = acétyle et/ou H, **caractérisé en ce que** du chlorhydrate de minocycline est transformé avec de l'anhydride acétique en présence d'un capteur de protons, dans lequel une acétylation unique ou multiple de la structure de commande s'effectue avec une élimination d'eau et aromatisation du cycle A simultanées, le produit de réaction est soumis à une purification chromatographique en utilisant un matériau support et un éluant, l'éluant est distillé et le produit est ensuite purifié par recristallisation, dans lequel au moins un R est = acétyle.

2. Procédé selon la revendication 1, **caractérisé en ce qu'**une base organique est utilisée comme capteur de protons.

3. Procédé selon les revendications 1 et 2, **caractérisé en ce que** la base est une amine primaire, secondaire ou tertiaire.

4. Procédé selon la revendication 1 ou 2, **caractérisé en ce que** le capteur de protons est de préférence une pyridine.

5. Procédé selon la revendication 1 ou 2, **caractérisé en ce que** l'anhydride acétique et le capteur de protons sont utilisés en excès ou de manière équimolaire en fonction du nombre des groupes acétyle à introduire.

6. Procédé selon une ou plusieurs des revendications précédentes, **caractérisé en ce que** la transformation des réactifs s'effectue dans un solvant inerte.

7. Procédé selon la revendication 6, **caractérisé en ce que** le solvant est le chloroforme, chlorure de méthylène, nitrométhane, acétonitrile, acétone, sulfolane, diméthylformamide ou diméthylsulfoxyde.

8. Procédé selon une ou plusieurs des revendications précédentes, **caractérisé en ce que** la transformation est réalisée à une température de 4 à 100°C à une pression normale ou surpression.

9. Procédé selon une ou plusieurs des revendications précédentes, **caractérisé en ce que** de la pentaacétylcycline (pentaacétyl-minocycline à cycle A aromatisé) de la Formule II 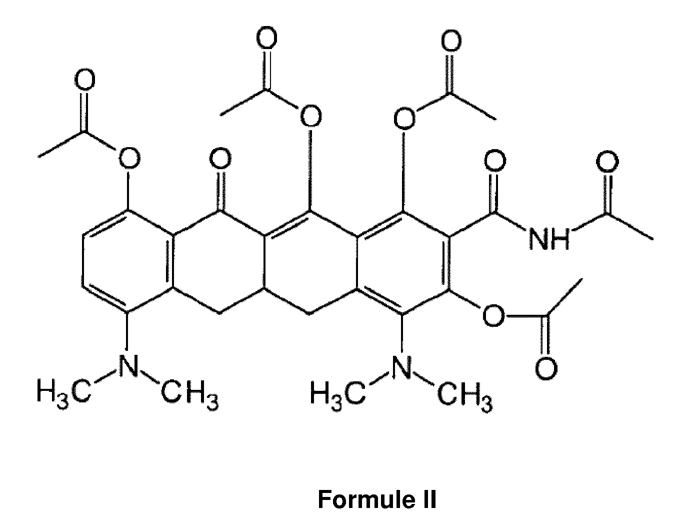 est synthétisée comme produit principal.

10. Procédé selon une ou plusieurs des revendications précédentes, **caractérisé en ce que** d'autres minocyclines à cycle A aromatisé, à acétylation unique ou multiple, de la Formule I (avec R¹ à R⁵ = acétyle et/ou H, dans lequel au moins un R = acétyle) sont synthétisées en fonction de la gestion de la réaction, dans lequel le nombre des groupes acétyle dans la molécule est inférieur ou égal à 5.

11. Procédé selon une ou plusieurs des revendications précédentes, **caractérisé en ce que** la tétraacétylcycline (tétraacétyl-minocycline à cycle A aromatisé) de la Formule IV 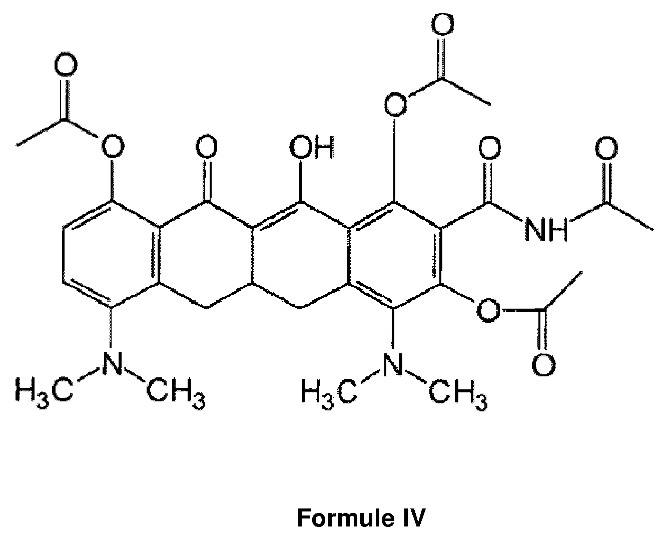 est synthétisée de manière particulièrement préférée comme produit secondaire.

12. Procédé selon une ou plusieurs des revendications précédentes, **caractérisé en ce que** la purification chromatographique des produits de réaction est réalisée sur du gel de silice 60 (gel de silice) ou un autre matériau support comme l'oxyde d'aluminium, du gel de silice 'à phase inversée' ou du Sephadex.

13. Procédé selon une ou plusieurs des revendications précédentes, **caractérisé en ce que** la purification chromatographique s'effectue en lit fixe, dans une colonne de chromatographie, dans un entonnoir Büchner avec insert de frittes de verre ou un récipient suspendu avec garniture à fond perforé.

14. Procédé selon une ou plusieurs des revendications précédentes, **caractérisé en ce qu'**un mélange d'acétate éthylique et d'essence de pétrole est utilisé de manière particulièrement préférée pour l'élution par le biais du matériau support et pour le recristallisation des produits de réaction.

15. Procédé selon une ou plusieurs des revendications précédentes, **caractérisé en ce que** du formiate méthylique, acétate *n*-butylique, diméthylcarbonate ou d'autres hydrocarbures comme le *n*-pentane, *n*-hexane, cyclohexane ou isobutane sont également utilisés à la place de l'acétate éthylique et de l'essence de pétrole.

16. Pentaacétylminocycline à cycle A aromatisé de la Formule II

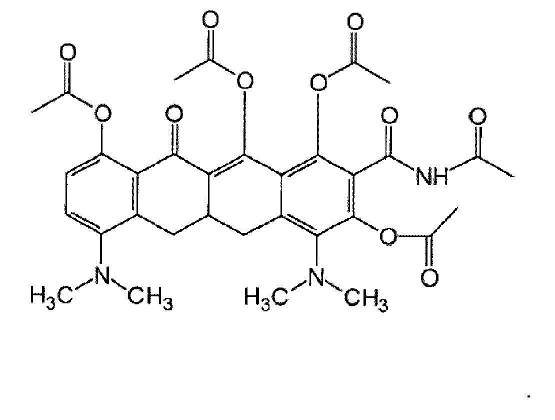

17. Tétraacétylminocycline à cycle A aromatisé de la Formule IV

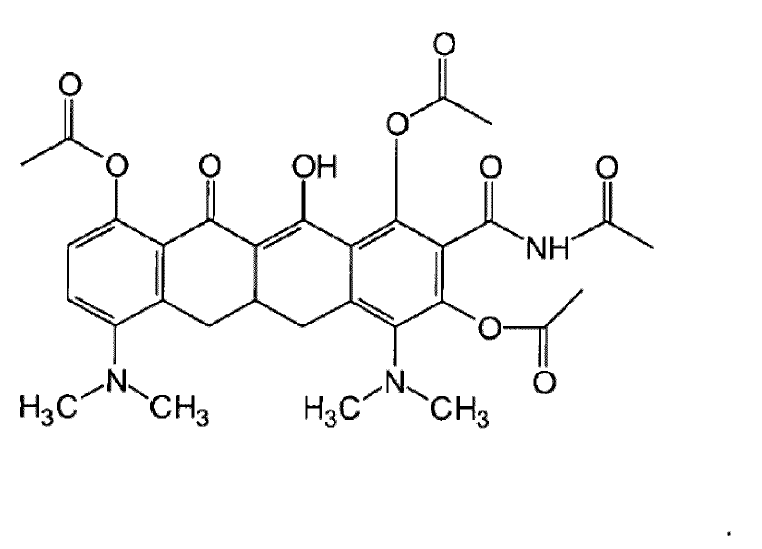

18. Utilisation de la substance active selon une des revendications 16 ou 17 pour la fabrication d'un médicament qui peut être utilisé pour le traitement de maladies neurodégénératives développées par stress oxydatif et/ou dommages mitochondriaux.
